# EUROPEAN PATENT APPLICATION

(11) **EP 1 321 117 A2**
(43) Date of publication of application: **25.06.2003**
(21) Application number: 02394118.0
(22) Date of filing: 19.12.2002
(51) Int. Cl.: A61F 7/00

(54) **Contrast bathing physiotherapy device**

(30) Priority: 19.12.2001 IE 20011081
(71) Applicant: FORFAS, (trading as PEI Technologies), Dublin 2 (IE)
(72) Inventor: Dalton, Tara, Castletroy, Limerick (IE); Punch, Jeff, County Limerick (IE)
(74) Representative: Weldon, Michael James

(57) **Abstract**

A contrast bathing physiotherapy device (1) has local thermoelectric cells (7) which heat or cool a localised part of a patient's body (B) via a Cu heat spreader 6 and a flexible thermal interface pad (15). Cooling is augmented by a bias bank (21) of thermoelectric cells which draw heat from the local bank (7) via a looped heat pipe (10). The heat pipe (10) has an evaporator in a Cu block (8) at the local bank (7) and a condenser in a Cu block (20) at the bias bank (21).

## Description

### INTRODUCTION

### Field of the Invention

The invention relates to physiotherapy devices or other therapeutic devices for heating or cooling part of a patient's body in a localised manner.

### Prior Art Discussion

Contrast bathing is a physiotherapy treatment where an injury is treated with alternate periods of hot and cold. This produces alternate dilations and contractions of the blood vessels, which aid in the absorption of swelling and thus promote the healing process.

It is known to provide a contrast bathing device comprising a heat exchange pad with circulation ducts for hot and cold liquids. The device also comprises a heater and a refrigeration unit for heating and cooling the liquids. A pad for such a device is described in United States Patent No. US4114620. Such devices therefore suffer from being bulky and expensive. Also, there is a slow response time for changing between heating and cooling modes.

Russian Patent Specification No. RU2084211 describes a thermoelectric physiotherapy apparatus, having a cooler in the form of an accumulator containing solid coolant such as ice. It appears that the apparatus would not work very effectively because it could not be operated continuously for long periods due to the use of an accumulator, a heat storage device with a finite capacity.

The invention is therefore directed towards providing a contrast bathing physiotherapy device with improved effectiveness for all modes of operation, namely heating, cooling, and contrast bathing.

### SUMMARY OF THE INVENTION

According to the invention, there is provides a contrast bathing physiotherapy apparatus comprising a controller and a thermoelectric cell heat transfer means, characterised in that,
the heat transfer means comprises a local thermoelectric cell connected to a thermal interface pad for contact with a patient's body, and a bias thermoelectric cell connected to the local bank and comprising means for augmenting the cooling effect of the local cell.

In one embodiment, the local and bias cells are interconnected by a looped heat pipe having a condenser section connected to the bias cell and an evaporator section connected to the local cell.

In another embodiment, the heat pipe is routed through a heat transfer block connected to the bias cell and a heat transfer block connected to the local cell.

In a further embodiment, the controller comprises means for directing current through the local cell in a direction to heat the thermal interface pad, and for allowing the local cell to heat the pad without assistance from the bias cell.

In one embodiment, the bias cell is connected to a heat sink mounted to transfer heat with the surrounding air whereby the surrounding air acts as a primary heat sink for the apparatus.

In another embodiment, the heat sink further comprises an air fan for circulation of air.

In a further embodiment, the controller comprises a temperature sensor mounted between a heat spreader block for the local bank and the thermal interface pad.

In one embodiment, the thermal interface pad is flexible.

In another embodiment, the thermal interface pad comprises metal particles embedded in a thermal paste.

In a further embodiment, the apparatus comprises a bank of local cells mounted in succession.

In one embodiment, the apparatus comprises a bank of bias cells mounted in successor.

### DETAILED DESCRIPTION OF THE INVENTION

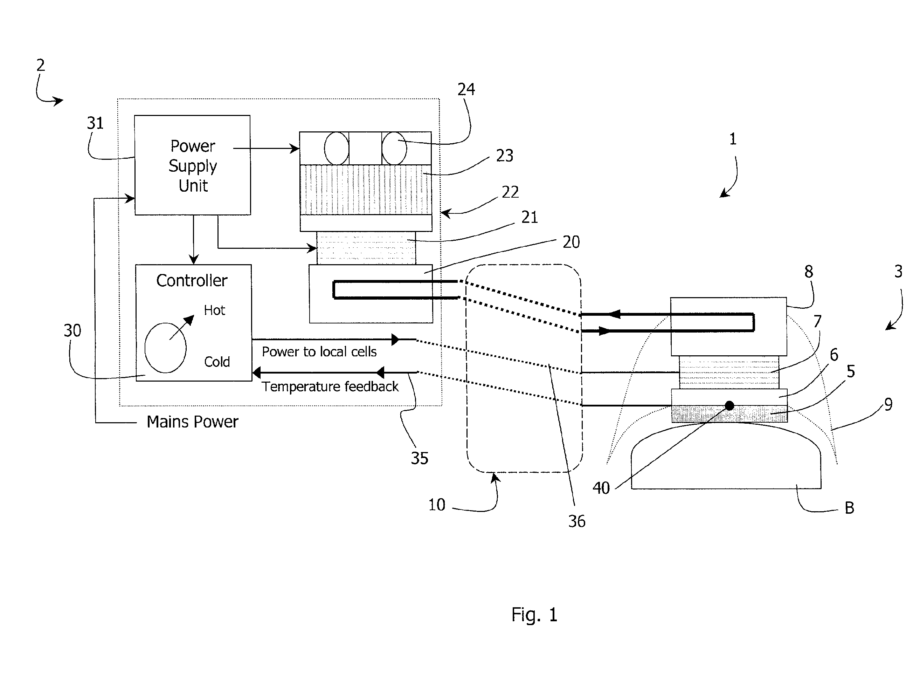

The invention will be more clearly understood from the following description of some embodiments thereof, given by way of example only with reference to the accompanying drawings in which:-
Fig. 1 is a schematic representation of a contrast bathing physiotherapy device of the invention.

Referring to Fig. 1 a contrast bathing physiotherapy device 1 comprises a control unit 2, and a local unit 3 for direct contact with the body B. The units 2 and 3 are interconnected by a looped heat pipe 10, a sealed assembly comprising two flexible pipes connecting an evaporator section and a condenser section. A looped heat pipe contains a working fluid such as water or alcohol, sealed at low pressure. A wick structure is present in the evaporator section in order to create capillary pressure. Heat applied to the evaporator section causes the working fluid to change phase from a liquid to a gas, and this gas is pumped by capillary pressure to the condenser section where it condenses, giving up heat and reverting to a liquid. The liquid is returned to the evaporator section by the capillary pressure.

The local unit 3 comprises, in sequence from the body B out:-
a thermal interface pad 5,
a copper heat spreader 6,
a bank of local thermo-electric (Peltier) cells 7, and
a Cu heat transfer block 8.

A shell structure and strapping are indicated by the numeral 9.

The control unit 2 comprises a Cu heat transfer bias block 20, and in order away from the block 20:
a bank of bias thermo-electric cells 21,
a Al heat sink 22 having fins 23, and
an air fan 24.

A controller 30 receives feedback of the temperature at the local unit on a signal line 35, and delivers power to the local cells 7 via a power cable 36. The controller 30 only controls the local cells, the bias cells being run at constant current from a power supply 31. The looped heat pipe 10 does not contain a pump - capillary action driving the heat pipe flow; and the fan 24 is run at constant RPM via a fixed voltage from the power supply 31.

Mains AC Power is drawn by the power supply unit 31 to provide DC outputs to the active components: the controller 30, the fan 24, and the bias cells 21.

The condenser and evaporator sections of the looped heat pipe 10 are located in the bias Cu block 20 and the local Cu block 8 respectively.

The thermal interface pad 5 comprises a malleable latex pouch containing a thermally-conductive mixture of Cu filings and thermal paste. A temperature sensor 40 for the signal line 35 is mounted between the heat spreader 6 and the pad 5.

Physically, the power supply unit 31, the controller 30, and the assembly of the bias cells 21, the heat sink 22 and the fans 24 are mounted within a single enclosure. The user controls are mounted on the face of the enclosure. The control unit and the bathing unit are connected by a flexible coupling containing the pipes of the looped heat pipe 10, the power line to the local cells, and the cables for the temperature feedback sensor. The local unit 3 comprises the local cells within its assembly and the thermal interface pad 5, mounted within a shell structure 9 which has strapping with Velcro™ fixings for attachment to the body.

In operation, the user sets the target temperature using the controller 30. The controller 30 compares the error between the target temperature and the signal from the temperature feedback sensor, and varies the current to the local cells 7 in order to minimise this error. To achieve a cool target temperature, the polarity of the current applied to the local cells 7 is set such that heat is extracted from the copper heat spreader 6 and the thermal interface pad 5, and pumped into the copper heat transfer block 8. To achieve a hot target temperature, the polarity of the current applied to the local cells 7 is reversed such that heat is extracted from the copper heat transfer block 8 and pumped into the copper heat spreader 6 and the thermal interface pad 5. For each case - cooling and heating - temperature control is achieved by varying the magnitude of the applied current. By this means, the bathing device can be used to apply a range of temperature treatments - heat, cold or contrast - to parts of the human body. In addition, a control algorithm of the controller 30 can be used to automatically cycle the target temperature from hot to cold for a pre-defined number of cycles of a specific period, or for a specified duration.

In more detail, the following describes operation for the different modes.

For both modes of operation - heating and cooling - the bias cells 21 operate at a fixed current with such bias that they pump heat from the copper heat transfer block 20 of the control unit into the heat sink 22. The fan 24 assists the transfer of this heat into the ambient air. The copper block 20 cools the condenser section of the looped heat pipe 10, cooling its evaporator section which, in turn, cools the copper heat transfer block 8 of the local unit. For both modes of operation, the function of the local cells 7 is different, as set out below.

Cooling: A current is applied to the local cells 7 such that their action is to pump heat from the copper heat spreader 5 and thermal interface block 5 into the copper heat transfer block 8. In this manner, the local cells 7 pump heat in the same direction as the bias cells 21. The heat transfer path is from the patient's body into the local block 8, through the heat pipe 10 to the bias block 20, and through the bias cells 21 to the surrounding air heat sink assisted by the fan 24. Thus the bias cells 21 act to extract heat from the local block 8. This is very advantageous because thermoelectric cells are inefficient. For example, 1W of heat extracted from the cold side of a cell gives rise to, for example, 2W pumped into its hot side. By pulling heat out of the local block 8 the bias cells are greatly assisting the cooling process.

Heating: A current is applied to the local cells 7 such that their action is to pump heat from the copper heat transfer block 8 into the copper heat spreader 5 and thermal interface block 5. In this manner, the local cells 7 pump heat in the opposite direction as the bias cells 21. In this mode, the inefficiency of a thermoelectric cell means that a relatively large quantity of heat is pumped into the hot side, in this case the pad 5. Thus, for example, for 2W of heat pumped into the pad 5 there may be only 1W extracted from the local block 8. The fact that the bias cells 21 also draw heat from the local block 8 means that they act counter to the local cells 7. However, this does not matter because of the small amounts of heat being drawn from the block 8 for a given level of heat on the pad 5. If this does become a problem for some embodiments having a large pad 5 then the bias cells 21 can simply be switched off.

In summary, for both modes, the controller 30 varies the magnitude of the current applied to the local cells 7 in proportion to the error between the target temperature and the signal from the temperature feedback sensor: the larger the error, the larger the current. The bias cells 21 always operate such that they cool the copper heat transfer block 8 of the local unit 3, irrespective of the bias of the current applied to the local cells 7. This is due to the poor efficiency of thermoelectric cells - for each Watt of heat extracted from the cool side of the cell, more than a Watt of heat is pumped to the hot side. Consequently, the cells are better heaters than coolers - hence the use of the bias cells to aid cooling.

The invention is not limited to the embodiments described but may be varied in construction and detail. For example, heat may be transferred between the local and bias cells by a steam/water pipe having a pump. Also, the bias cells and other components of the control unit may be located in the same physical enclosure as the local cells.

## Claims

1. A contrast bathing physiotherapy apparatus (1) comprising a controller (30) and a thermoelectric cell heat transfer means, **characterised in that**,
the heat transfer means comprises a local thermoelectric cell (7) connected to a thermal interface pad (5) for contact with a patient's body, and a bias thermoelectric cell (21) connected to the local bank (7) and comprising means for augmenting the cooling effect of the local cell (7).

2. A contrast bathing physiotherapy apparatus as claimed in claim 1, wherein the local and bias cells (7, 21) are interconnected by a looped heat pipe (10) having a condenser section (20) connected to the bias cell and an evaporator section (8) connected to the local cell (7).

3. A contrast bathing physiotherapy apparatus as claimed in claim 2, wherein the heat pipe (10) is routed through a heat transfer block (20) connected to the bias cell (21) and a heat transfer block (8) connected to the local cell (7).

4. A contrast bathing physiotherapy apparatus as claimed in any preceding claim, wherein the controller (30) comprises means for directing current through the local cell in a direction to heat the thermal interface pad (5), and for allowing the local cell (7) to heat the pad (5) without assistance from the bias cell (21).

5. A contrast bathing physiotherapy apparatus as claimed in any preceding claim, wherein the bias cell (21) is connected to a heat sink (22) mounted to transfer heat with the surrounding air whereby the surrounding air acts as a primary heat sink for the apparatus.

6. A contrast bathing physiotherapy apparatus as claimed in claim 5, wherein the heat sink (22) further comprises an air fan (24) for circulation of air.

7. A contrast bathing physiotherapy apparatus as claimed in any preceding claim, wherein the controller (30) comprises a temperature sensor mounted between a heat spreader block (6) for the local bank (7) and the thermal interface pad (5).

8. A contrast bathing physiotherapy apparatus as claimed in any preceding claim, wherein the thermal interface pad (5) is flexible.

9. A contrast bathing physiotherapy apparatus as claimed in claim 8, wherein the thermal interface pad (5) comprises metal particles embedded in a thermal paste.

10. A contrast bathing physiotherapy apparatus as claimed in any preceding claim, wherein the apparatus comprises a bank of local cells mounted in succession.

11. A contrast bathing physiotherapy apparatus as claimed in any preceding claim, wherein the apparatus comprises a bank of bias cells mounted in successor.
